Europäisches Patentamt

European Patent Office

Office européen des brevets

(19)

(11) Publication number: **0 154 392**

**A2**

(12) # EUROPEAN PATENT APPLICATION

(21) Application number: 85300369.7

(22) Date of filing: 21.01.85

(51) Int. Cl.⁴: **G 01 N 33/576**
**G 01 N 33/567**

(30) Priority: 02.03.84 US 585806

(43) Date of publication of application:
11.09.85 Bulletin 85/37

(84) Designated Contracting States:
BE CH DE FR GB IT LI NL SE

(71) Applicant: WARNER-LAMBERT COMPANY
201 Tabor Road
Morris Plains New Jersey 07950(US)

(72) Inventor: Burk, Kenneth H.
13428 Hedgeapple Drive
Dallas Texas 75243(US)

(74) Representative: Jones, Michael Raymond et al,
HASELTINE LAKE & CO. Hazlitt House 28 Southampton
Buildings Chancery Lane
London WC2A 1AT(GB)

(54) Processes for the detection of non-A, non-B hepatitis and a kit for use in the same.

(57) There is disclosed a process for assaying a tissue specimen for non-A, non-B, hepatitis antigen. The process comprises:

(a) providing a tissue specimen to be assayed;

(b) contacting the tissue specimen with a non-A, non-B hepatitis antibody;

(c) contacting the resultant tissue specimen with a biotin-avidin system; and

(d) determining the presence of non-A, non-B hepatitis antigen present in the tissue sample.

Also described in an analogous process for assaying for non-A, non-B hepatitis antibody in a biological sample.

A diagnostic kit and a fixed, embedded tissue sample are also disclosed.

EP 0 154 392 A2

PROCESSES FOR THE DETECTION OF NON-A, NON-B HEPATITIS
AND A KIT FOR USE IN THE SAME

The present invention relates to a method for the detection of non-A, non-B hepatitis agents and in particular to a method of detecting non-A, non-B hepatitis agents using a tissue screen technique. The tissue screen technique uses an enhanced identification method which renders the inventive procedure a useful diagnostic testing system.

Non-A, non-B hepatitis (NANB) has been recognized as a virus-induced disease, distinct from other forms of viral-associated liver diseases, including hepatitis A (HAV) and B (HBV), and the hepatitides induced by cytomegalovirus (CMV) or Epstein-Barr virus (EBV). Several attempts have been made to identify specific NANB antigen-antibody reactions using a variety of methods. To date, neither specific non-A, non-B antigen nor non-A, non-B antibody have been identified. Similarly a number of NANB associated ultra-structural changes and virus-like particles have been described. However, the NANB virus has not yet been definitively identified.

U.S. Patent 4,356,164 to Tabor describes a method of assaying for the amount of non-A, non-B hepatitis antigen present in a sample comprising a first step of reacting antigen in the sample with the antibody, a second step of separating reactive phase from non-reactive phase by precipitating antibody from serum and purifying said antibody, and a third step of measuring the extent of the reaction as a measurement of the amount of non-A, non-B hepatitis antigen present in the sample. Tabor purports to disclose the identification of non-A, non-B hepatitis antigen using counterelectrophoresis (CEP) which Tabor also describes as immunoelectrosmophoresis (IEOP) or immunoelectrodiffusion (IED) or countercurrentelectrophoresis.

The techniques involved testing sera using 1%

2

agarose in barbital buffer, pH 8.6, poured onto 3.5 x 12.5 cm glass plates. Melted agarose was then poured onto a lantern slide. When it had cooled, two rows of holes were punched in the agarose. Antibody was placed in one row of holes and samples to be tested were added to the other row. When testing for antibody, antigen was added to one row and samples in the other row. The lantern slide was placed in a CEP chamber. Paper wicks were used to connect each side of the slide to each of two pools of barbital buffer, pH 8.6. An electric current was passed across the plate, 35 milliamps per plate, for one hour. Immunoprecipitin lines were read after 1, 24, and 48 hours of storage in a moist chamber at room temperature. When the test sample was positive, a precipitin line was seen between the rows, using the naked eye with the aid of an electric lamp. The diagnostic technique described is extremely time consuming, subject to erroneous interpretation and does not necessarily represent an antigen-antibody reaction.

In contrast, International Patent Application No. PCT/US82100166 to Maupas discloses the isolation from serum of two non-A, non-B hepatitis virus particles.

Maupas describes the first particle as resembling a togavirus. It is a spherical, coated virus having an outer diameter of 50-60 nm and a core having a diameter of about 40 nm. Heating an aqueous suspension of this virus at 25° C for extensive periods or exposing the virus to ether causes it to lose its infectivity for tissue culture. It was recovered from the plasma or urine of patients suffering from NANB hepatitis.

The second particle Maupas describes resembles an enterovirus. This enterovirus-like particle is spherical, having a diameter of about 27 nm; exhibits a buoyant density of 1.36 in isopyenic density-gradient centrifugation with cesium chloride; loses its tissue culture infectivity upon heating in aqueous suspension for 3 hours at 90° C, but not after 10 hours at 56° C; it is insensitive to ether; and its genetic material is positive to a histochemical stain for RNA.

Both particles have been cultured in vitro in tissue culture.

Even with the prior art developmental activities, a need still exists for a sensitive, specific method for screening and identification of NANB hepatitis. NANB infection has been implicated in 60-90% of post-transfusion hepatitis cases. The risk of contracting NANB hepatitis after blood transfusion has been estimated to be close to 10% in the U.S. Therefore, it is important to screen the blood of potential donors to detect NANB hepatitis. Further, based upon studies in chimpanzees, the chronic sequelae associated with NANB-hepatitis now appear to be more serious than chronic disease developments arising from HBV infection.

There has been discovered an unexpected process which uses a tissue screen method for determining the presence of non-A, non-B hepatitis agents and particularly antibodies and antigens, which is extremely sensitive.

More particularly the invention involves a process for assaying a tissue specimen for non-A, non-B hepatitis antigen by obtaining a tissue specimen which may contain non-A, non-B hepatitis antigen, contacting the tissue specimen with a non-A, non-B antibody, contacting the resultant tissue specimen with a biotin - avidin system, and determining the presence of non-A, non-B hepatitis antigen present in the tissue sample.

In another embodiment the invention involves a process for assaying for non-A, non-B hepatitis antibody present in a biological sample by contacting a tissue specimen containing non-A, non-B hepatitis

4

antigen with the biological specimen to permit reaction of the specific specimen antibodies with the non-A, non-B hepatitis antigen, incubating the reactants for up to 24 hours at temperatures up to 45° C, separating the reactive tissue specimen from the non-reactive phase, contacting the resultant tissue specimen with a biotin-avidin enhancement system, and determining the presence of non-A, non-B hepatitis antibody in the sample.

An additional embodiment involves a process for assaying for non-A, non-B hepatitis antigen present in a biological sample, by reacting antigen present in the sample with non-A, non-B hepatitis antibody, separating reactive phase from non-reactive phase by precipitating immune-complexed antibody antigen and recover the fluid supernatant, contacting a tissue specimen containing non-A, non-B antigen with the fluid supernatant to permit reaction of any non precipitated non-A, non-B hepatitis antibodies with the non-A, non-B hepatitis antigen, incubating the reactants for up to 24 hours at temperatures up to 45° C, contacting the resultant tissue specimen with a biotin - avidin system, and determining the presence of non-A, non-B hepatitis antigen in the sample.

The present test method is extremely useful in screening patients serum prior to use of the blood for transfusion purposes; diagnosis of non-A, non-B hepatitis infections; detection of non-A, non-B hepatitis antigen and antibody in biological samples; and liver tissue screening for the presence of antigen. Such procedures have been heretofore not possible with previously reported techniques for detection of low levels of non-A, non-B hepatitis antigen and antibody.

As used herein, the term "biotin-avidin" system refers to a particular enhancement marker for the identification of the presence or absence of a

particular analyte, such as antigen or antibody. Such systems are generally well known and do not constitute an inventive aspect of this invention other than by their critical use to detect NANB agents. Such techniques have been known for detecting herpes simplex virus, see Nerurkar et al (1983) J Clin Microbiol "Detection of Genital Herpes Simplex Infections by a Tissue Culture fluorescent - Antibody Technique with Biotin-Avidin" (17)1: 149-154; papillomavirus, see Mounts et al (1982) Proc Natl Acad Sci USA "Viral etiology of juvenile and adult - onset of squamous popilloma of the larynx" (79)17:5425-5429; and cytomegalovirus antigens, see Oefinger et al (1983) American Society of Microbiology "Characterization of the humoral response of BALB/C mice immunized with human cytomegatovirus," 83rd Annual Meeting; March 6-11, 1983 Orleans, LA p. 73.

A patented process is described by Hevey et al. in U.S. Patent No. 4,228,237, the process described involves a process for detection and determination of a liquid which employs an insoluble phase enzyme immunoassay wherein an enzyme labeled avidin and a biotin labeled reagent detect the liquid.

In addition, Parith, U.S. Patent No. 4,298,685 discloses a process for the detection and/or determination of a biological substance in a test sample, which comprises admixing the test sample, a predetermined quantity of a soluble enzyme-label for of the biological substance, and a predetermined quantity of a soluble biotin tagged antibody raised against the biological substance. The reactants are allowed to come to equilibrium and then an insolubilized avidin is added. The resulting solid phase is separated from the liquid phase and the enzyme activity of either of these phases is then determined.

Any of these enhancement systems are useable in the present invention.

As used herein, the term "non-A, non-B hepatitis" refers to a biologically active material which is distinct from hepatitis A and hepatitis B virus. The non-A, non-B hepatitis agents of this invention do not react immunologically with tests for either of the known viruses.

The present inventors have identified a distinct non-A, non-B virus material. This material has not been able to be grown in tissue culture and is not present in stool as previously reported by Maupas.

The term "kit" as employed herein means a collection of all or some of the chemicals necessary to do an immunoassay. A diagnostic kit has been developed which contains a tissue specimen containing non-A, non-B hepatitis antigen. Tissue source may be selected from liver tissue containing active virus activity.

Tissue preservation may be performed by cutting a source of tissue containing the non-A, non-B hepatitis antigen into convenient sizes. Tissue preservation may be performed by standard fixation and embedding techniques. Such procedures may employ formalin and/or aldehydes as the fixing agent with formaldehyde and glutaraldehyde being preferred aldehydes and paraffin as an embedding agent. Other standard materials may also be employed within the scope of the invention.

Alternatively the tissue may be preserved by standard cryopreservation techniques wherein the tissue is unfixed and frozen. Prior to preservation the tissue may be conveniently cut into cubes or wedges having preferred sizes of 1 cubic centimeter or less.

Once inactivated the tissue is embedded and stored for future use. The tissues may then be cut into 2 to 6 micron thick sections and stored for future use.

The tissue sections are now ready for use. The tissue sections may then be analyzed for the presence or absence of non-A, non-B hepatitis antigen. One procedure for performing this analysis involves

7

contacting the tissue specimen with a non-A, non-B antibody, contacting the resultant tissue specimen with an identification system, and determining the presence of non-A, non-B hepatitis antigen present in the tissue sample. This technique is a simple and convenient method for identifying the presence of non-A, non-B hepatitis antigen in infected liver.

To limit non-specific reaction of the biological specimen antibodies to binding sites in the tissue specimen other than non-A, non-B hepatitis antigen, the tissue may be pretreated with a blocking agent. Exemplary blocking agents may be selected from a wide range of material including normal IgG, normal IgM, bovine serum albumin and normal animal serum. The blocking agent may be used in amounts of about 1% to about 25% and preferably 5% to 12% by weight of a buffered solution. Blocking may be conveniently performed by treating the tissue specimen with the blocking agent at room temperature for up to 1 hour and then removing excess blocking agent with buffered solution. The buffered solutions used herein are not critical with preferred buffers having neutral pH and low ionic strength such as phosphate buffered saline pH 7.0-7.5 (0.01 molar phosphate; 0.15 molar saline).

The tissue specimen is then contacted with the biological specimen suspected of containing non-A, non-B hepatitis antibody. The antibody may be derived from normally induced human antibody, chimpanzee antibody and other non human primate antibody. In addition, antibody may be derived from animal immunization resulting in polyclonal and/or monoclonal non-A, non-B hepatitis antibody. Contacting may be performed at temperatures up to 45° C for times up to 24 hours and preferably 20 to 37°C for 2 to 24 hours.

The identification system used in the present invention must be sufficiently sensitive to detect the

NANB tissue antigens. It has been found that use of an avidin-biotinylated enzyme complex is at least 40-fold more sensitive than two conventional immunoperoxidase techniques according to dilutional sensitivity studies performed with HBV tissue antigens.

Utilization of the avidin/biotin amplified immunoperoxidase staining procedure, in conjunction with a primary convalescent antiserum obtained from a NANB-hepatitis donor, resulted in the observation of NANB virus associated antigen in the hepatocytes of an infected chimpanzee liver. The same human antiserum was not reactive with a number of uninfected control cells, nor with cells infected with hepatitis A virus, hepatitis B virus or cytomegalovirus. Preincubation of this convalescent NANB serum or IgG derived thereof, with an antigenemic serum obtained from a NANB infected chimpanzee, abolished the antibody reactivity. This is also true in alternate systems which employ animal antibody with animal antigen such as human antibody and human antigen, chimpanzee antibody and chimpanzee or human antigen.

According to the invention, the resultant tissue specimen can then be contacted with the biotin labeled antibody and then contacted with an avidin-biotin system with readable label. The procedure for performing is reaction falls within the scope defined by the prior art and is not critical other than order in which the tagging is used.

Exemplary procedures may employ any non-competitive binding process such as the "Sandwich" technique. In this method the components of the binding reaction may comprise the insoluble tissue specimen containing NANB antigen for the NANB antibody and the following reagents:

(i)  a measured amount of liquid medium suspected of containing NANB antibody;

(ii)    biotin labeled specific binding
        substance for anti NANB antibody;

(iii)   avidin; and

(iv)    biotinylated-labeled enzyme.

When the insoluble phase is incubated with individual reagents in particular sequence, the binding process can be affected in either two or three incubation periods.

In a three step incubation process, the insoluble phase is first reacted with the liquid reagent i.e., a measured amount of liquid medium suspected of containing the NANB antibody. Unreacted reagent is then removed and biotin labeled specific binding substance for NANB antibody is added. Following the reaction of the biotin labeled specific binding substance, unreacted biotin labeled reagent is removed and enzyme labeled avidin is added. Following the reaction of enzyme labeled avidin, unreacted reagent is separated from the insoluble phase and the enzyme activity of either the insoluble phase or the separated unreacted enzyme labeled avidin is determined by a suitable detection reaction. The enzymatic activity of the insoluble phase is directly related to the amount of ligand in the liquid medium whereas the enzymatic activity of separated unreacted enzyme labeled avidin is inversely related in the amount of ligand.

When biotin labeled specific binding substance is prereacted with enzyme labeled avidin the binding process can be affected in two steps by substituting the biotin labeled reagent bound to enzyme labeled avidin for the individual biotin labeled and enzyme labeled avidin reagents.

Biotin-tagged antibody is conveniently prepared by reaction of a biotin derivative, for instance a biotin ester derivative such as the N-hydroxysuccinimide ester of biotin, with the antibody. The biotin ester derivative is dissolved in a polar, aprotic solvent, for example dimethylformamide, and is then added in a 20 to 300 molar excess to the antibody in 0.01 M to 1.0 M preferably 0.05 M to 0.5 M, phosphate buffer. The phosphate buffer may have a pH 6.5 to pH 8.5.

After admixture of the reactants, the reaction is allowed to proceed at a temperature of from 2° to 10° C for a time sufficient for its completion. Normally this takes about 10 hours. After completion of the reaction, the biotin-tagged antibody may be separated from the reaction mixture by standard methods well known in the art, for example by gel permeation chromatography on, for instance, a crosslinked dextran.

The enzyme labeled avidin reagent or biotin reagent of the instant invention is prepared by conventional procedures, such as by covalent coupling of the selected enzyme to avidin or biotin. This covalent linking can be achieved by direct coupling of existing linking groups or by the addition of external bridging molecules. When using horseradish peroxidase blocking of amino groups and hydroxy groups with fluorodmitrobenzene prior to oxidation prevents self-coupling and allows the enzyme to condense with available amino groups present in avidin. Moreover, alkaline phosphatase can be coupled to avidin or biotin by the condensation of free amino groups in the foregoing molecules with aldehyde groups provided by an external bridging molecule such as glutaraldehyde and other polyfunctional bridging compounds.

Although a wide variety of enzymes can be used to prepare the enzyme labeled avidin or biotin reagent, exemplary enzymes include oxidoreductases and hydrolases according to the International Union of Biochemists (I.U.B.) Particularly preferred oxidoreductases include, glucose oxidase and horseradish peroxidase. Particularly preferred hydrolases include, for example, alkaline phosphatase and B-galactosidase.

The determination of enzyme activity in the instant invention may, for example, be accomplished with a visual detection or microscopic detection of the reaction system suitable for the particular enzyme employed.

A suitable method for assaying for non-A, non-B hepatitis antibody present in a biological sample, would involve contacting a tissue specimen suspected of containing non-A, non-B antigen with the biological specimen to permit reaction of the specimen non-A, non-B hepatitis antibodies with the non-A, non-B antigen. If specific antibodies were present binding would occur at the NANB antigen site. The reaction is optimized by incubating the reactants for up to 24 hours at temperatures up to 45° C. Once the reaction is complete the reactive tissue specimen is separated from the non-reactive phase and the resultant tissue specimen is contacted with a biotin-avidin enhancement system as described above.

The presence or absence of non-A, non-B hepatitis antibody in the sample may then be made by visual observation in a microscope. A positive result indicates the presence of non-A, non-B hepatitis antibody in the sample.

A suitable method for assaying for non-A, non-B hepatitis antigen present in a biological sample, would involve reacting suspected NANB antigen present in the sample with a known non-A, non-B hepatitis antibody for up to 10 days at temperatures up to 45°C. This reaction may also be performed in two steps. The first step preferably having a higher temperature and shorter reaction time than the second step. A preferred first step could be performed at a temperature from 20 to 45°C for 1 to 5 hours. This first step aids in the formation of the antibody-antigen complex by driving the reaction to the right, that is to complex formation. The second step is then performed to complete the reaction and form a precipitable immune complex. The second reaction step is performed at a lower temperature than the first step for a longer period of time. Useable temperatures may be from 2°C to 15°C for times up to 15 days. Once the reaction is complete the reactive phase is separated from non-reactive phase by precipitating immune-complex antibody - antigen and recovering the fluid supernatant. The immune-complex may be precipitated by standard techniques such as centrifugation or reaction with precipitating agents, for example with second antibody, polyethyleneglycol and so forth.

A tissue specimen containing non-A, non-B antigen is then contacted with the fluid supernatant to permit reaction of any non-precipitated non-A, non-B hepatitis antibodies with the non-A, non-B insoluble tissue hepatitis antigen. The reactants are preferably incubated for up to 24 hours at temperatures up to 45° C.

The resultant tissue specimen is then contacted with a biotin - avidin enhancement system described above. The presence or absence of non-A, non-B hepatitis antigen in the sample may then be made by visual observation in a microscope. A negative result indicates the presence of non-A, non-B hepatitis antigen in the sample.

This invention also comtemplates a kit for assaying a specimen for a non-A, non-B hepatitis agent which may contain all or some of the materials including a positive tissue sample containing non-A, non-B hepatitis antigen. Preferable the kit contains a tissue specimen which may be fixed and embedded with the non-A, non-B hepatitis agent.

The kit may additionally contain one or more of the following: a quantity of non-A, non-B antibody; a quantity of biotinylated antibody against said non-A, non-B antibody which is derived from a host distinct from the host used to produce the non-A, non-B antibody;

a quantity of avidin and/or biotinylated linked enzyme, and a substrate for the determination of the enzyme activity of said enzyme.

The present invention is further illustrated by the following examples. All parts and percentages in the examples as well as in the specification and claims are by weight unless otherwise specified.

EXAMPLES

Chimpanzee Materials

Liver biopsy material was obtained from chimpanzees infected with human plasma containing infectious NANB hepatitis virus. Liver wedge biopsies were obtained from each of the animals at a time during which liver enzyme profiles were elevated and pathological indicators of viral hepatitis were noted. Sera were collected from each of these animals prior to experimental infection with NANB virus, as well as at weekly intervals after infection for documentation of liver enzyme changes, and establishment of the acute disease phase. In addition, sera were also procured from a number of 2-3 year old juvenile chimpanzees, for use as negative controls.

Human Materials

Liver tissue was obtained at autopsy from a chronic carrier of HBV. The histopathology of the specimen was consistent with a diagnosis of chronic hepatitis with cirrhosis. Immunocytochemical techniques had previously documented the presence of HBsAg and HBcAg in the hepatocytes of this tissue. Normal control liver tissue was procured which had no serological markers of HBV infection and the tissue contained no detectable HBsAg or HBcAg by immunocytochemical analysis.

Sera were obtained from several human donors for these immunocytochemical staining studies. A serum was obtained from a patient who had recovered from NANB implicated viral hepatitis approximately 7 months after his liver enzyme profile had returned to normal and during which time no clinical symptoms indicative of disease were noted. Sera were absorbed with acetone extracts of liver powder in order to eliminate nonspecific immunocytochemical reactions. Human or canine liver powders are rehydrated in phosphate buffered saline, and incubated with an equal volume of test serum for one hour. After a second adsorption, the serum was clarified by centrifugation at 3000 rpm and filtered through a 0.22 micron filter.

Two human antisera were utilized in a study designed to compare the relative sensitivities of three different staining techniques for the detection of HBcAg and HBsAg in a formalin-fixed and paraffin-embedded liver tissue described below. The first human serum was obtained from an HBV chronic carrier with antibody to HBcAg (anti-HBc) as detected by solid phase RIA, but with no detectable antibody to HBsAg (anti-HBs). The second serum was taken from a hemopheliac who had circulating antibody to both HBsAg and HBcAg. Additional normal control sera were obtained from donors who had no serological or clinical evidence of infection with HAV, HBV, or NANB virus.

Tissue Preservation

Fresh human and chimpanzee liver tissues were cut into 1 cm cubes, washed in cold phosphate buffered saline (PBS), pH 7.2 and fixed 18-24 hours in 10% buffered formalin at room temperature. The tissues were dehydrated through a graded ethanol series, through toluene, and finally infiltrated and embedded in paraffin, as described previously. The paraffin-embedded tissues were then cut in 4-5 micron thick sections on a microtome.

Immunocytochemistry

Three immunocytochemical procedures were evaluated as described below.

1.   Indirect Immunoperoxidase (IP)

An indirect immunoperoxidase procedure was performed with the immunoconjugate of goat anti-human. IgG-horseradish peroxidase (HRPO) used as a second antibody which was diluted 1:10 in a solution of 10% normal goat serum in PBS (10% NgtS-PBS).

2.    Staphylococcal Protein-A Peroxidase-anti-per<del>Oxi</del>8<del>4</del>892
      (Staph A-PAP)

The staph A-PAP procedure employed was originally described by Notani et al (1979) J Histochem Cytochem "Versatility of Staphylococcus Aureus Protein A in Immunocytochemistry."27:1138-1444 which procedure used a rabbit peroxidaseantiperoxidase.

3.    Avidin-Biotin Immunoperoxidase (ABC-IP)

The avidin-biotin-immunocytochemical procedure was performed according to general method described by Hsu et al (1981) J. Histochem Cytochem "Use of avidin-biotin-peroxidase complex (ABC) in immunoperoxidase techniques."    (29)4:577-580.  A secondary biotinylated goat anti-human IgG and an avidin-biotinylated horseradish peroxidase complex (ABC) was used.

The procedure involved taking tissue sections which were pretreated with 10% NgtS-PBS, drained, and incubated with the individual diluted chimpanzee or human sera.  All primary sera were diluted in 10% NgtS-PBS, and incubations were maintained at room temperature throughout the procedure.  After washing with cold PBS, the tissue sections were next incubated with an optimal dilution (1:400 - 1:800 in 10% NgtS-PBS) of biotinylated goat anti-human IgG.  After a 30 minute incubation, the sections were again washed, and incubated with the ABC complex.  Excess reagents were removed from the tissue sections by 3 washes with 0.05M tris HCl pH 7.6 (Tris buffer).  The liver sections were then reacted in the dark with a substrate solution containing 0.5 mg/ml diaminobenzidine-$(HCl)_4$, containing 0.01% $H_2O_2$ in Tris buffer.  Selected specimens were also counterstained with hemotoxylin. The sections were further dehydrated in ethanol, transferred into toluene and mounted.

Titration Studies

Reactive antibody from human donor serum (heat inactivated at 56° C for 60 minutes) was titrated by serial two-fold dilution from 1:2 to 1:128 in normal human serum, and screened against NANB-infected chimpanzee liver by the ABC-IP procedure. All serum dilutions were screened for the presence of IgG antibody as well as IgM antibody by reacting with biotinylated goat anti-human IgM.

To confirm the antibody activity observed in the donor serum, purified IgG from donor serum was screened and titrated as above. Purification of the globulins was accomplished by addition of $(NH_4)_2SO_4$, and the IgG fraction was further purified by DEAE cellulose ion exchange chromatography.

Blocking Studies

Reactive antibody samples were screened again by the ABC-IP procedure following pre-incubation with acute phase serum from chimpanzees or from human patients infected with NANB hepatitis in order to immunologically block the antibody activity. Immunological blocking was evidenced by a considerable diminution or complete elimination of the immunoperoxidase staining. Sample aliquots of donor serum (100 ul) were incubated with an equal volume of presumably antigenemic serum (chimp or human) for 2 hours at 37C, followed by an overnight incubation at 4°C. At the end of the second incubation all samples were centrifuged at 5,000 rpm for 30 minutes at 4°C and the supernatant serum protein titrated for residual antibody activity. Control samples for the blocking studies included normal human serum, young (2-3 yr.) juvenile chimpanzee serum, chimpanzee pre-inoculation sera, and heat inactivated (56°C, 1 hour) acute phase chimpanzee serum.

Relative Sensitivities of Immunocytochemical Methods

HBV reagents, i.e., human sera containing antibody to HBV antigens of a HBV antigen positive human liver, were utilized to evaluate the relative sensitivity levels of the three immunocytochemical staining procedures. The three techniques used were the IP, the Staph A-PAP and the ABC-IP. The two human sera materials described containing either antibody to HBcAg only or antibody to both HBcAg and HBsAg, were tested undiluted and at 2-fold dilutions with tissue sections obtained from a chronic HBV infected liver, and with tissue from a normal, uninfected human liver. No staining of the infected liver tissue section was noted using either sera as primary reagents in the IP procedure. However, a weak reaction was noted with both sera in the nuclei of isolated hepatocytes with the staph A-PAP method at sera dilutions of 1:2 and 1:4 in the absence of cytoplasmic staining. The same reagents were also used to test the sensitivity of the ABC-IP staining procedure. At a serum dilution of 1:80 both sera stained the nuclei of the majority of cells in isolated hepatic lobules. The human serum obtained from a hemophiliac patient which contained both anti-HBc and anti-HBs activity, also stained the cytoplasm of isolated hepatocytes as well as cell nuclei at a serum dilution of 1:40. Neither serum stained liver tissue procured from an individual negative for HBV serologic markers using the ABC-IP method. In addition, serum obtained from a control individual was negative for anti-HBs and anti-HBc failed to react with the infected liver tissue.

## Screening of NANB Infected Liver With Anti-NANB Serum

Liver tissue sections were procured from chimpanzees with pathological evidence for NANB hepatitis infection. These tissues were screened against heat treated donor serum by the ABC-IP immunocytochemical procedure for anti-NANB/NANB antigen reaction. The ABC-IP procedure yielded consistent, positive evidence for cytoplasmic antigen reactivity. Intense cytoplasmic staining was visible in 7-10% of the hepatocytes. Control liver specimens obtained from normal (i.e., non-infected) chimpanzee and reacted with heat treated donor serum were consistently negative for cytoplasmic staining. Similarly, normal human liver, HBV-infected human liver, and HAV-infected chimpanzee liver were all negative for cytoplasmic staining when reacted with donor serum. As a final control, normal human serum was unreactive with NANB-infected chimpanzee liver. The cytoplasmic localization of the specific stain reaction was clearly demonstrated. Adsorption of donor serum with canine or normal human liver powder did not diminish the immunoreaction with the NANBinfected chimpanzee liver.

Titration of Antibody Activity to NANB Antigen

An aliquot of several donor serum, procured approximately seven months into convalescence (i.e., after all chemical and clinical symptoms returned to normal), was titered by serial two-fold dilutions from 1:2 through 1:128 and screened against NANBinfected chimpanzee liver. Positive staining was observed in the cytoplasm and scored as either intense (++), moderate (+), or negative (-). The serum titer of anti-NANB was 1:32, with moderate staining observed (Table I). In a second group of titration experiments, IgG, derived from donor serum, was diluted as described above and screened against NANB-infected chimpanzee liver. An endpoint titer of 1:16 was observed when scored as above (Table I).

## TABLE I

### Titration of Antibody Activity To NANB Antigen

| Antibody Reagent | Un-dilute | 1:2 | 1:4 | 1:8 | 1:16 | 1:32 | 1:64 | 1:128 |
|---|---|---|---|---|---|---|---|---|
| Donor Serum Antibody #1 | ++ | ++ | ++ | ++ | ++ | + | – | – |
| Donor Purified IgG #1 | ++ | ++ | ++ | + | + | – | – | – |
| Donor Serum Antibody #2 | ++ | ++ | ++ | + | – | – | – | – |
| Donor Serum Antibody #3 | ++ | ++ | ++ | ++ | ++ | ++ | + | – |
| Donor Serum Antibody #4 | ++ | ++ | ++ | + | – | – | – | – |

Blocking Studies

The specificity of the observed cytoplasmic stain reaction was investigated by immunological blocking of donor serum antibody activity with chimpanzee or human implicated NANB acute and chronic sera. Donor serum stain activity was not blocked by pre-incubation with either preinoculation chimpanzee serum, or normal human serum, normal chimpanzee serum, or young juvenile chimpanzee serum. However, preincubation of donor serum with either of two acute phase NANB chimpanzee sera or with chronic phase NANB human serum, effectively blocked antibody activity. In addition, antibody activity in purified donor IgG was blocked with these antigenemic sera. Results of all blocking studies are summarized in Table II.

### TABLE II

| Antibody pre-incubated with serum from: | Residual actibody reactivity in: | |
|---|---|---|
| | Serum | IgG |
| Normal human | ++ | ++ |
| Normal chimp | ++ | ++ |
| Juvenile chimp | ++ | ++ |
| Pre-inoculation chimp[b] | ++ | ++ |
| Acute chimp A[b] | - | - |
| Acute chimp B[b] | - | - |
| Chronic human[b] | - | - |

Cytoplasmic Staining was scored as intense (++), moderate (+), or negative (-). The b. notation indicates serum obtained from chimpanzees before and after inoculations with NANB or from a NANB infected human.

A summary of the above staining patterns was demonstrated by a number of control experiments. The results indicated that donor serum did not react with liver tissue from normal uninfected chimpanzee or human. In addition, no reaction was noted when the same serum was used to stain sections of HAV- or HBV-infected liver tissue. Normal chimpanzee or human serum did not stain the NANB-infected tissue by the ABC-IP method. The most convincing evidence for specificity was obtained by preincubation of donor serum with either of two different sera from two chimpanzees derived during the acute phase of their NANB infection. Preincubation with either of these two presumably antigenemic sera completely blocked the staining reaction, whereas, preincubation with normal chimpanzee or human serum had no observable effect on the intensity of the staining. These results indicated the specificity of the ABC-IP reaction for NANB associated antigens.

The invention being thus described, it will be obvious that the same may be varied in many ways. Such variations are not to be regarded as a departure from the spirit of scope of the invention and all such modifications are intended to be included within the scope of the claims.

-23- 0154392

CLAIMS:

1. A process for assaying a tissue specimen for non-A, non-B hepatitis antigen, which process comprises:

(a) providing a tissue specimen to be assayed;

(b) contacting the tissue specimen with a non-A, non-B hepatitis antibody;

(c) contacting the resultant tissue specimen with a biotin-avidin system; and

(d) determining the presence of non-A, non-B hepatitis antigen present in the tissue sample.

2. A process according to Claim 1, wherein the tissue specimen is a piece of liver tissue.

3. A process according to Claim 2, wherein the tissue specimen is fixed and embedded.

4. A process according to Claim 2, whrein the tissue specimen is unfixed, frozen tissue.

5. A process according to any preceding claim, wherein the tissue specimen is pretreated or otherwise contacted with a blocking agent to block nonspecific binding sites, the blocking agent preferably being chosen from: normal IgG, normal IgM, bovine serum albumin, and normal animal serum.

6. A process according to any preceding claim, wherein the non-A, non-B hepatitis antibody is IgG or IgM.

7. A process according to any preceding claim, wherein the tissue specimen is contacted with the non-A, non-B hepatitis antibody at a temperature of up to 45°C, preferably from 20°C to 37°C, for a time of up to 24 hours, preferably from 2 to 24 hours.

8. A process according to any preceding claim, wherein the non-A, non-B hepatitis antibody

0154392

of step (b) is biotinylated prior to its contacting with the tissue specimen.

9. A process according to any preceding claim, wherein the resultant tissue specimen of step (c) is contacted with a biotin labelled antibody against the non-A, non-B hepatitis antibody which biotin labelled antibody is derived from a host distinct from the host sued to produce the non-A, non-B hepatitis antibody.

10. A process according to any one of Claims 1 to 7, wherein the resultant tissue specimen of step (c) is first contacted with a biotin labelled antibody and then contacted with readable labelled avidin-biotin system.

11. A process for assaying for non-A, non-B hepatitis antibody present in a biological sample, which process comprises:

(a) contacting a tissue specimen containing non-A, non-B hepatitis antigen with the biological specimen to permit reaction of the specimen non-A, non-B hepatitis antibodies with the non-A, non-B hepatitis antigen;

(b) incubating the reactants for a time of up to 24 hours at a temperature of up to 45°C;

(c) separating the tissue specimen from the non-reactive phase;

(d) contacting the resultant tissue specimen with a biotin-avidin system; and

(e) determining the presence of non-A, non-B hepatitis antibody in the sample.

12. A process according to Claim 11, wherein a positive result indicates the presence of non-A, non-B hepatitis antibody in the sample.

13. A process for assaying for non-A, non-B hepatitis anitgen present in a biological sample, which process comprises:

(a) reacting antigen present in the sample

-25-  Q154392

with non-A, non-B hepatitis antibody for a time of up to 10 days at a temperature of up to 45°C;

(b)  separating reactive phase from non-reactive phase by precipitating immune-complexed antibody - antigen and recovering the liquid supernatant;

(c)  contacting a tissue specimen containing non-A, non-B hepatitis antigen with the liquid supernatant to permit reaction of any non-precipitated non-A, non-B hepatitis antibodies with the non-A, non-B hepatitis antigen present in the tissue specimen;

(d)  incubating the reactants for a period of up to 24 hours at a temperature of up to 45°C;

(e)  contacting the resultant tissue specimen with a biotin-avidin system; and

(f)  determining the presence of non-A, non-B hepatitis antigen in the sample.

14.  A process according to Claim 13, wherein a negative result indicates the presence of non-A, non-B hepatitis antigen in the sample.

15.  A process according to Claim 13 or 14, wherein the reaction of anitgen with antibody in step (a) is performed in two steps, the first step being at a temperature of from 20 to 45°C for from 1 to 5 hours and a second step at a temperature lower than the temperature of the first step and for a time longer than that of the first step.

16.  A tissue specimen which is fixed and embedded and which contains non-A, non-B hepatitis antigen.

17.  A diagnostic kit which comprises:

(a)  a tissue specimen which is fixed and embedded and which specimen contains non-A, non-B hepatitis antigen;

(b)  a quantity of non-A, non-B hepatitis antibody;

(c)   a quantitiy of a biotin-avidin system; and

(d)   a substrate for the determination of the enzyme activity of the enzyme.

18.   A diagnostic kit according to Claim 17, wherein the biotin-avidin system comprises a biotin labelled antibody and a readable labelled avidin-biotin system.